# Europäisches Patentamt

(19) **European Patent Office**

**Office européen des brevets**

(11) Veröffentlichungsnummer: **0 078 961**

**B1**

(12) **EUROPÄISCHE PATENTSCHRIFT**

(45) Veröffentlichungstag der Patentschrift:
15.10.86

(51) Int. Cl.⁴: **A 61 K 37/14**

(21) Anmeldenummer: **82109808.4**

(22) Anmeldetag: **23.10.82**

(54) Verfahren zur Herstellung eines lagerstabilen, vernetzten Hämoglobinpräparates mit hoher Sauerstoff-Transportkapazität, sowie das nach diesem Verfahren hergestellte Hämoglobinpräparat.

(30) Priorität: **11.11.81 DE 3144705**

(43) Veröffentlichungstag der Anmeldung:
**18.05.83 Patentblatt 83/20**

(45) Bekanntmachung des Hinweises auf die Patenterteilung:
**15.10.86 Patentblatt 86/42**

(84) Benannte Vertragsstaaten:
**AT BE CH DE FR GB IT LI LU NL SE**

(56) Entgegenhaltungen:
**DE-A-2 449 885**
**DE-A-2 617 822**
**DE-A-2 714 252**

**J. Surg. Res. 30(1981), 14-20**

**Die Akte enthält technische Angaben, die nach dem Eingang der Anmeldung eingereicht wurden und die nicht in dieser Patentschrift enthalten sind.**

(73) Patentinhaber: **Biotest- Serum- Institut GmbH, Flughafenstrasse 4, D-6000 Frankfurt- Niederrad (DE)**

(72) Erfinder: **Bonhard, Klaus, Dr., Dipl.- Chem., Georg- Wolff- Strasse 5, D-6450 Hanau 1 (DE)**
Erfinder: **Kothe, Norbert, Dr., Dipl.- Chem., Friedrich- Ebert- Strasse 21, D-6242 Kronberg/Ts. (DE)**

(74) Vertreter: **Beil, Walter, Dr., BEIL, WOLFF & BEIL Rechtsanwälte Adelonstrasse 58, D-6230 Frankfurt am Main 80 (DE)**

**Beschreibung**

Die Erfindung betrifft ein Verfahren zur Herstellung eines lagerstabilen, vernetzten Hämoglobinpräparates mit hoher Sauerstofftransportkapazität, bei dem man eine stromafreie Hämoglobinlösung deoxygeniert und mit einem Effektor, einem carbonylgruppen-spezifischen Reduktionsmittel und bei einem pH-Wert zwischen 6 und 8 mit Dialdehyden mit Kohlenstoffketten mit 3 bis 8 C-Atomen versetzt, sowie das nach diesem Verfahren hergestellte Hämoglobinpräparat.

Bis in die erste Hälfte dieses Jahrhunderts reichen Bemühungen zurück, Hämoglobinlösungen als "Blutersatz" zu verwenden. Die Entwicklung begann damit, daß man versuchte, Erythrozyten-Hämolysate zu infundieren. Es stellte sich jedoch schnell heraus, daß die in diesen Präparaten enthaltenen Stromabestandteile toxisch auf die Nieren wirkten, und außerdem das Gerinnungssystem beeinflußten.

Die Entwicklung ging nun dahin, daß man versuchte, stromafreie, Hämoglobinlösungen herzustellung. Dies gelang auf zwei unterschiedlichen Wegen. Durch Zentrifugation und Ultrafiltration konnten Rabiner et al. 1967 erstmals eine stromafreie Lösung herstellen. (Rabiner S.F. et al., J.ExP.Med., 126 1127 (1967). Diese Methode wurde von K.Bonhard weiterentwickelt. (DE-PS 2 248 475). In letzter Zeit wurde über ein kristallisiertes stromafreies Hämoglobin berichtet (De Venuto F. et al., J.Lab.Clin.Med. 89, 509 (1977). Diese stromafreien Hämoglobinlösungen sind jedoch als "Blutersatz" ungeeignet, da sie einige entscheidende Nachteile aufweisen.

Der intraerythrozytäre pH-Wert liegt niedriger als derjenige des Plasmas (ca. 7,2 gegenüber 7,4). Diese pH-Wert-Differenz bewirkt eine Linksverschiebung der Sauerstoffbindungskurve, also eine Erhöhung der Sauerstoffaffinität, noch verstärkt durch Entzug des Effektors 2,3-Diphosphoglycerat (DPG), der im Erythrozyten die Sauerstoffabgabe reguliert. Nach den beschriebenen, Verfahren hergestellte Hämoglobinlösungen weisen im Kreislauf nur einen Halbsättigungsdruck ($p_{50}$-Wert) von ca 16 mbar auf. In vitro gelingt es zwar, den $p_{50}$-Wert durch Zusatz von 2,3-DPG auf 31 mbar zu erhöhen, aufgrund der schwachen Bindung des DPG an das Hämoglobin wird der Effektor in vivo jedoch sehr rasch wieder durch die Niere ausgeschieden. Dieser Nachteil konnte durch Verwendung eines fester gebundenen Effektors eliminiert werden. In der DE-OS 26 17 822 ist ein Verfahren beschrieben, das unter Verwendung von Pyridoxalphosphat zu einem Hämoglobinpräparat mit erhöhter Sauerstoffabgabe führt.

Obwohl das Problem der geringen Sauerstoffabgabe gelöst werden konnte, blieb ein entscheidender Nachteil der beschriebenen Lösungen bestehen, nämlich daß die intravasale Halbwertszeit dieser Lösungen nur ca. 100 Minuten, beträgt. Das im Plasma gelöste Hämoglobin wird aufgrund seiner Struktur und seiner Größe (Molekulargewicht 64500 D) rasch durch die Niere eliminiert und beeinflußt außerdem, wenn es in so großen Mengen, wie es für einen Blutersatz notwendig ist, infundiert wird, vorübergehend die Nierenfunktion.

Zur Verlängerung der intravasalen Halbwertszeit wurden verschiedene Verfahren angewandt, die alle zum Ziel hatten, das Molekulargewicht des Hämoglobins zu vergrössern. Es wurde versucht, Hämoglobin mit anderen makromolekularen Polymeren wie Dextran (Chang J.E. et al., Can.J.Biochem., 55, 398 (1977), Hydroxyäthylstärke (DE-OS 26 16 086), Gelatine (DE-AS 24 49 885), Albumin (DE-AS 24 49 885) und Polyethylenglykol (DE-OS 30 26 398) zu verknüpfen. Es wurden ferner die verschiedensten Vernetzungsmittel eingesetzt, um die Hämoglobinmoleküle untereinander zu verknüpfen (DE-AS 24 49 885, US-PS 4 001 200, US-PS 4 001 401).

Gemäß einer Ausführungsform der DE-AS 24 49 885 werden die vernetzten Produkte anschließend mit einem Effektor wie Pyridoxalphosphat behandelt.

Aus der DE-OS 27 14 252 ist ein Verfahren gemäß dem Oberbegriff des Anspruchs 1 bekannt. Durch die dort beschriebene Behandlung in der Reihenfolge Effektor → Reduktion mit einem carboxylgruppenspezifischen Reduktionsmittel → Dialdehyd erhält man Produkte, bei denen der Dialdehyd nicht ausreichend fixiert ist.

Wie alle anderen vorstehend beschriebenen vernetzten Präparate weisen die letztgenannten Produkte zwar eine verlängerte intravasale Halbwertszeit auf, sind jedoch mit weiteren verschiedenen anderen Nachteilen behaftet.

Durch die Verknüpfung der Hb-Moleküle untereinander oder mit anderen Makromolekülen durch bivalente Reagentien entstehen keine Produkte mit einheitlichem Molekulargewicht, sondern disperse Systeme mit sehr breiten Molekulargewichtsverteilungen. Diese reichen vom vierkettigen Grundmolekül über Oligomere davon bis hin zu hochpolymerisierten Molekülen. Welchen Einfluß eine solch breite Molekulargewichtsverteilung auf die Verträglichkeit dieser Lösungen hat, ist bisher noch nicht ganz geklärt, es wird jedoch diskutiert, daß histologische Veränderungen an Nieren und Leber von Versuchstieren auf Polymere bzw. monomere Anteile zurückzuführen sind.

Chemische Modifizierungen am Hämoglobinmolekül verändern häufig die Sauerstoffbindungskurve. In den meisten Fällen wird die Affinität zum Sauerstoff erhöht, so daß unter physiologischen Bedingungen weniger Sauerstoff an das Gewebe abgegeben wird. In anderen Fällen wird das chemisch veränderte Hämoglobin unter dem Sauerstoff-Partialdruck der Lunge (ca. 133 mbar) nur noch wenig mit Sauerstoff beladen, wodurch ein ausreichender Sauerstofftransport wiederum nicht gewährleistet ist. Die Bindung von Effektoren wie Pyridoxalphosphat vor der Vernetzung wirkt zwar dem Anstieg der Sauerstoffaffinität entgegen, ein $p_{50}$-Wert oberhalb 27 mbar beim plasmat. PH von 7,4 konnte jedoch nie reproduzierbar erhalten werden.

Die Vernetzung setzt zugleich auch die Lagerstabilität der Hb-Lösungen stark herab. Limitierender Faktor der Lagerungszeit ist die Bildung von Methämoglobin, das keinen Sauerstoff transportiert und die Lösung allmählich unwirksam macht.

2

Der Erfindung lag die Aufgabe zugrunde, ein vernetztes, also eine verlängerte intravasale Halbwertszeit aufweisendes Hämoglobinpräparat herzustellen, das

a) eine enge Molekulargewichtsverteilung aufweist, d.h. frei von hochpolymerisiertem Hämoglobin und arm an unvernetztem Hämoglobin ist,

b) diese stabile Molekulargewichtsverteilung beibehält und trotz der Vernetzung

c) eine hohe Sauerstofftransportfähigkeit und

d) gute Lagerstabilität aufweist.

Diese Aufgabe wird erfindungsgemäß dadurch gelöst, daß man vor dem Zusatz des Effektors eine stromafreie angereicherte Hämoglobinlösung mit soviel eines Sauerstoff verbrauchenden Reduktionsmittels versetzt, daß der $O_2$-Partialdruck auf 0 mbar sinkt, das nach Zusatz des Effektors erzielte Produkt anschließend mit dem Dialdehyd versetzt, das dadurch vernetzte Produkt mit der 5- bis 20-fachen Menge des carbonylgruppen-spezifischen Reduktionsmittels reduziert, die Lösung mit Wasser verdünnt, ggf. mit Aktivkohle behandelt, solange im Kreislauf ultrafiltriert bis der Gehalt an unvernetztem Hämoglobin höchstens noch 15 % beträgt, wobei die Vernetzung vor oder während der Ultrafiltration erfolgen kann, und das Produkt durch Zugabe eines Reduktionsmittels stabilisiert.

Die erfindungsgemäß hergestellten Hämoglobinpräparate weisen keinen der vorher beschriebenen Nachteile auf.

Die vorstehend genannten Eigenschaften a) bis d) werden im wesentlichen durch folgende Stufen des Gesamtverfahrens erzielt:

a) durch Kreislaufultrafiltration bis zu einem Gehalt an unvernetztem Hämoglobin von höchstens 15 %;

b) durch Reduktion mit einem carbonylgruppen-spezifischen Reduktionsmittel;

c) durch völlige Desoxygenierung auf chemischem Wege mit einem Sauerstoff verbrauchenden Reduktionsmittel und

d) durch Zusatz von reduzierenden Substanzen zum vernetzten, ultrafiltrierten Hämoglobinpräparat.

Die Vernetzung erfolgt mit Dialdehyden, die Kohlenstoffketten mit 3 bis 8 C-Atomen aufweisen, wie beispielsweise in der DE-AS 24 49 885 beschrieben.

Um die Sauerstoffaffinität zu verringern erfolgt vor der Vernetzung eine Behandlung mit einem Physiologisch verträglichen Effektor, beispielsweise wie in der DE-OS 26 17 822 beschrieben mit Pyridoxalphosphat oder mit Inosithexaphosphat, wie in der DE-OS 27 40 053 beschrieben.

Nach diesen beschriebenen Verfahren hergestellte Lösungen besitzen noch eine erhöhte Sauerstoffaffinität ($p_{50}$-Wert ca. 20 mbar).

Bei einer Ausführungsform des in der DE-AS 24 49 885 beschriebenen Vernetzungsverfahrens und gemäß dem Verfahren der DE-OS 27 14 252 erfolgt zwar auch vor der Vernetzung bzw. vor der Effektorbehandlung eine Überführung der Hämoglobinlösungen in die Desoxyform, jedoch findet die Desoxygenierung dort wie bisher üblich durch Evakuieren und Belüften mit Stickstoff statt. Dabei gelingt jedoch bei größeren Mengen keine vollständige Desoxygenierung. Bei einem Versuch, nach diesem Verfahren 2 l 18%ige Hämoglobinlösung in einem 5 l Gefäß zu desoxygenieren, fiel z.B. der $O_2$-Partialdruck selbst nach 40maliger Wiederholung nur auf 33 mbar ab. Aus der Sauerstoffbindungskurve ist zu entnehmen, daß das Hämoglobin bei diesem $O_2$-Partialdruck immer noch zu 75% gesättigt ist.

Überraschenderweise wurde gefunden, daß man ein mit einem Effektor, wie Pyridoxalphosphat, behandeltes vernetztes, reduziertes und ultrafiltriertes Hämoglobinpräparat mit einem $p_{50}$-Wert von 33-38 mbar bei PH 7,4 herstellen kann, wenn man die Hämoglobinlösung vor der Vernetzung vollständig, nämlich auf chemischem Wege in die Desoxyform überführt. Eine völlige Desoxygenierung auf chemischem Wege war bisher nicht bekannt.

Es war lediglich bekannt, in Hämoglobinlösungen enthaltenes Methämoglobin mit Hilfe von Ascorbinsäure zu reduzieren. Wollte man derart behandelte Produkte desoxygenieren, erfolgte anschließend eine Stickstoffbehandlung.

Erfindungsgemäß wird der Sauerstoff durch ein Sauerstoff verbrauchendes Reduktionsmittel, wie beispielsweise Ascorbinsäure, reduziertes Glutathion oder reduziertes Methylenblau, vorzugsweise neutralisierte Ascorbinsäure, aus der Lösung quantitativ entfernt. Die Effektivität des Verfahrens ist aus Tabelle 1 zu ersehen.

**Tabelle 1**

| Mol mit NaOH neutralisierte Ascorbinsäure / mol Hb | $pO_2$ (mbar) |
|---|---|
| 0 | 263 |
| 0,5 | 71 |
| 1 | 48 |
| 2 | 31 |
| 3 | 17 |
| 4 | 0 |

3

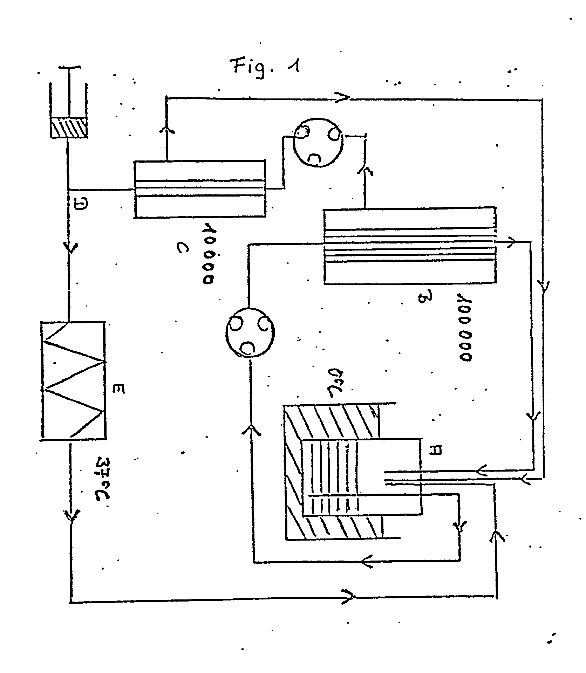

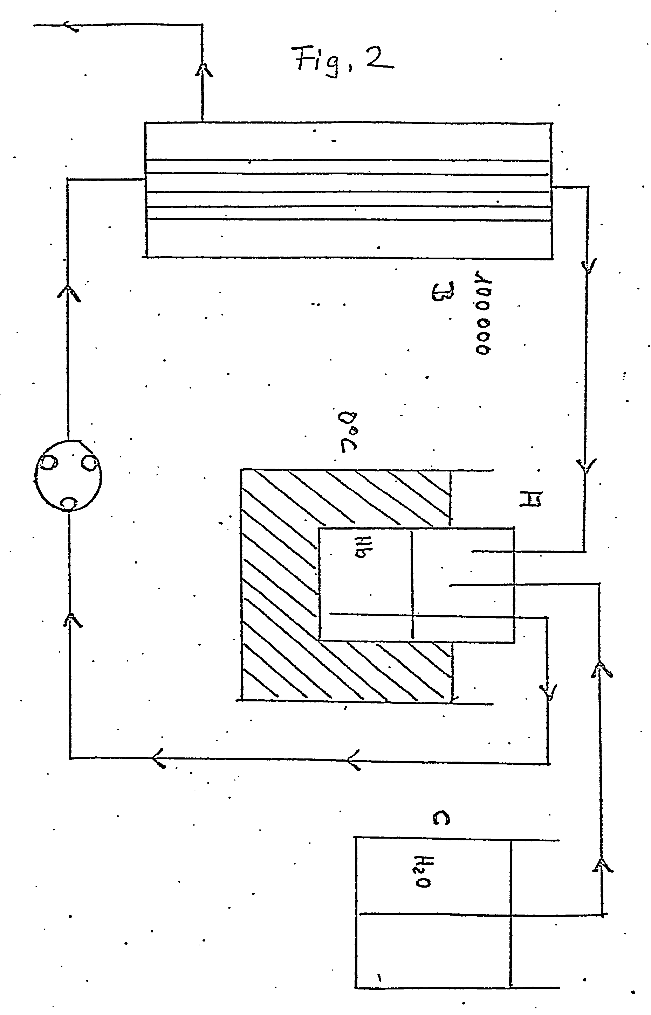

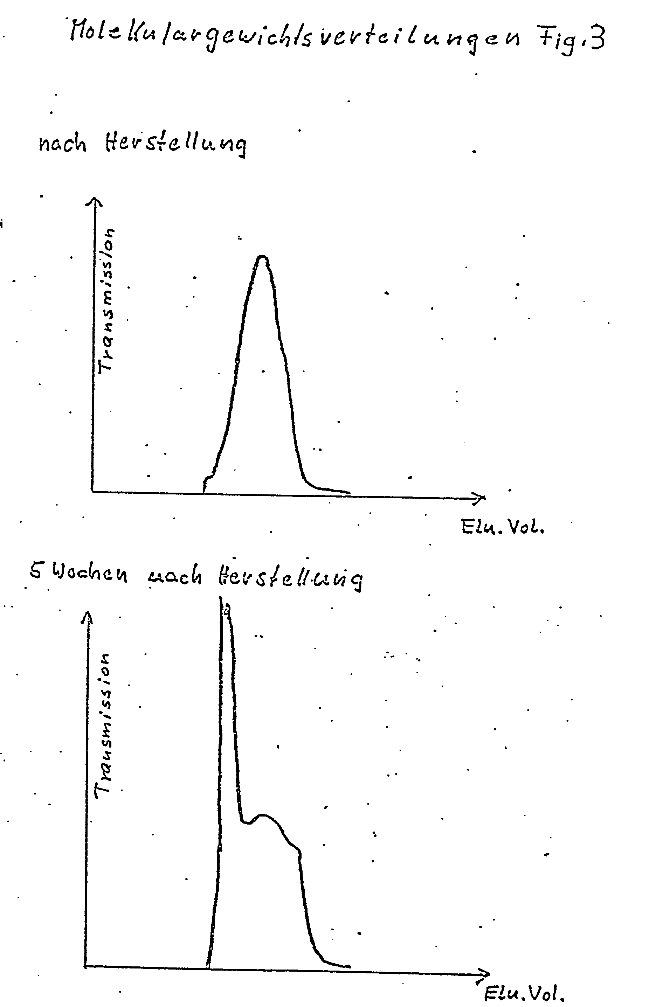

Die oben beschriebenen vernetzten Hämoglobinlösungen haben noch den Nachteil, daß die Stabilität der Molekulargewichtsverteilung zeitlich begrenzt ist. Im Laufe der Lagerung findet eine Verschiebung zu höheren Molekulargewichten statt (Fig. 3). Die Reaktion von Aldehyden mit Aminogruppen zu Azomethinen erfolgt sehr rasch, ist also kinetisch kontrolliert. Die Azomethinbindung jedoch ist leich hydrolytisch spaltbar, so daß sich während der Lagerung thermodynamisch stabilere Verknüpfungen durch Umlagerung der Azomethinbindung bilden können. Um dies zu verhindern, wird die Azomethinbindung durch Reduktion mit einem carbonylgruppen-spezifischen Reduktionsmittel stabilisiert, wie beispielsweise $NaBH_4$, $KBH_4$, $NaCNBH_3$ und katalytische Hydrierung.

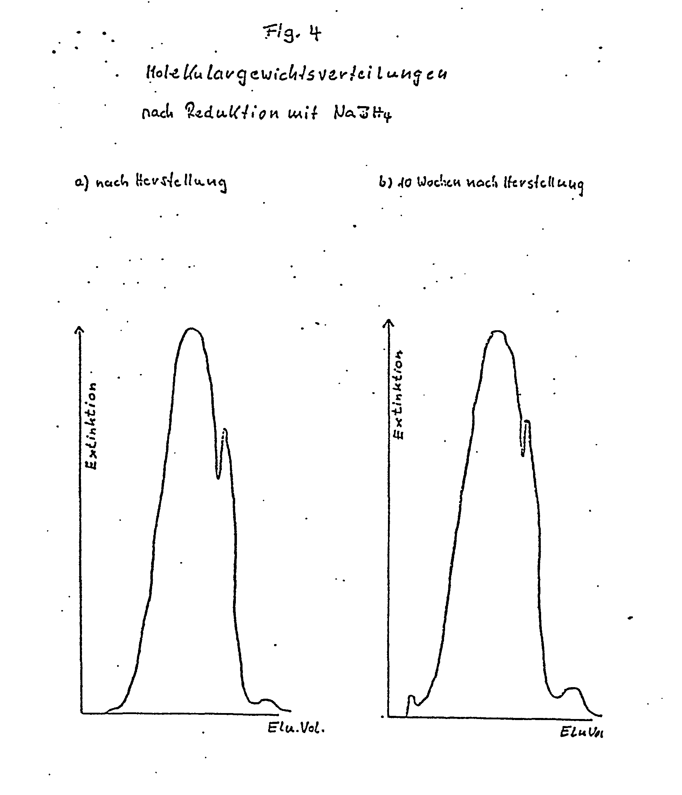

In Fig. 4 ist ein Beispiel der Reduktion mit $NaBH_4$ dargestellt. Das bei der Reaktion entstehende $BO_3^{3-}$ wird bei der Ultrafiltration entfernt.

Einen weiteren Vorteil bietet das Reduktionsverfahren, wenn man Pyridoxalphosphat als Effektormolekül verwendet. Auch das Pyridoxalphosphat ist über eine Azomethinbindung an das Hämoglobinmolekül gebunden. Mit Hilfe der Reduktion werden also nicht nur die Quervernetzungen stabilsiert, sondern auch der Effektor an seiner spezifischen Bindungsstelle fixiert.

Die zur Erzielung eines vernetzten Hämoglobin-Derivates, das frei von polymerisiertem Hämoglobin und möglichst arm an unvernetztem Hämoglobin ist, verwendete Ultrafiltrationsanlage besteht vorzugsweise aus Hollow-Fiber-Patronen. Man kann jedoch auch Flachmembranfilter verwenden.

Die Ultrafiltration kann nach den zwei nachfolgend beschriebenen Systemen erfolgen.

**System 1**

(Fig. 1)

Im gekühlten Vorratsgefäß A wird eine desoxygenierte, mit Effektor versetzte, unvernetzte Hämoglobinlösung vorgelegt und durch die Hollow-Fiber-Eiheit B mit einer Durchlässigkeit von Molekulargewicht 100 000 im Kreislauf gepumpt. Das Ultrafiltrat wird mit Hilfe einer zweiten Hollow-Fiber-Einheit (C) der Durchlässigkeit 10 000 ankonzentriert, bei D mit dem Vernetzungsmittel vermischt, im Reaktionsbad E auf 37°C erwärmt und in das Vorratsgefäß A zurückgeleitet. Die vernetzten Moleküle können die Membran der Ultrafiltrations-Einheit B nicht mehr Passieren. Mit dieser Anordnung ist gewährleistet, daß schon vernetztes Hämoglobin nicht ein zweites Mal mit Vernetzungsmittel in Berührung kommen kann, die Bildung von Polymeren also verhindert wird. Der Rest an unvernetzt gebliebenem Hämoglobin kann nach Beendigung der Reaktion durch Ultrafiltration entfernt werden.

**System 2**

(Fig. 2)

Der zweiten Möglichkeit, zu einem mehr einheitlich vernetzten Produkt zu gelangen, liegt folgende Überlegung zugrunde. Bei der Vernetzungsreaktion konkurrieren inter- und intramolekulare Vernetzung. Durch Erhöhung der Hämoglobinkonzentration läßt sich die Reaktion zugunsten der intermolekularen Vernetzung verschieben. Wählt man nun die Vernetzungsmittelmenge so, daß gerade kein polymeres Produkt entsteht, so erhält man neben einer gewissen Menge unvernetztem Hämoglobin nur Oligomere. Die unvernetzten Hämoglobinmoleküle werden in einem sich anschließenden Ultrafiltrationsschritt an einer Membran mit cut-off bei MG 100 000 teilweise abgetrennt. Die Vernetzungsreaktion erfolgt im Reaktionsgefäß A. Nach Beendigung der Reaktion wird die Lösung im Kreislauf durch die Ultrafiltrationseinheit B gepumpt, wobei unvernetztes Hämoglobin die Membran passiert. Der Volumenverlust im Kreislauf, bedingt durch das Abfließen des Ultrafiltrats, wird aus dem Vorratsgefäß C durch Wasser ersetzt.

Nach diesen Verfahren hergestellte vernetzte, oligomere Hämoglobinlösungen (Mn ca. 200 000 D) enthalten keine Polymeren Hämoglobine und weniger als 15 % unvernetztes Hämoglobin.

Während in System 1 von unvernetzten Hämoglobinlösungen ausgegangen wird und die Vernetzung während des Ultrafiltrationskreislaufs erfolgt, der so gesteuert ist, daß keine polymeren Produkte entstehen, werden gemäß System 2 vernetzte Produkte mit einer bereits gesteuerten Vernetzung eingesetzt.

Im Anschluß an die Reaktion mit dem carbonylgruppenspezifischen Reaktionsmittel wird mit Wasser verdünnt, damit der Schaum der stark schäumenden Lösung zusammenfällt.

Zur Reduzierung lipophiler Substanzen und zur Verbesserung der Filtrierbarkeit kann die verdünnte Lösung gegebenenfalls mit Aktivkohle behandelt werden.

Das erfindungsgemäße Gesamtverfahren bei Anwändung des Systems 2 läuft folgendermaßen ab.

Die 15 bis 30%ige Ausgangshämoglobinlösung wird je nach $O_2$-Partialdruck mit soviel eines Sauerstoff verbrauchenden Reduktionsmittels, zweckmäßigerweise mit NaOH neutralisierte Ascorbinsäure, versetzt, daß der $O_2$-Partialdruck auf 0 mbar absinkt. Bei einem mit $O_2$ gesättigten Produkt werden dies 4 Mol Ascorbinsäure je Mol Hb sein. Die auf 0 bis 10°C gekühlte Lösung wird mit der 2- bis 5-fach molaren, vorzugsweise mit der 3,35fach molaren Menge eines Effektors wie z.B. Pyridoxalphosphat oder Inosithexaphosphat versetzt. Nun erfolgt die Vernetzung mit dem Dialdehyd bei pH 6 bis 8, vorzugsweise 7,0, in vorzugsweise 10%iger wäßriger Lösung, wobei die Menge so dosiert wird, daß kein polymeres Hämoglobin entsteht.

Die anschließende Reduktion wird mit der 5 bis 20fach; vorzugsweise 13,8 fach molaren Menge des carbonylgruppenspezifischen Reduktionsmittels, z.B. $NaBH_4$, gemessen am Ausgangshämoglobin, durchgeführt.

Nun wird so lange durch ein Filter mit einer Durchlässigkeit bis MG 100 000 D ultrafiltriert, bis der Anteil vom unvernetzten am gesamten Hämoglobin im Unfiltrat zwischen 5 und 15% beträgt.

Die Lagerstabilität von vernetzten Hämoglobinlösungen ist durch die Bildung von Methämoglobin begrenzt. Erfindungsgemäß werden diese Hämoglobinpräparate durch Zusatz von reduzierenden Substanzen wie Ascorbinsäure, reduziertes Glutathion oder reduziertes Methylenblau gegen die Autoxydation stabilisiert. Nach einer Lagerzeit von 1 Jahr bleibt der Methämoglobingehalt dann noch unter 10 % des Gesamthämoglobins. Dabei ist es unerheblich, welchen Effektor man benutzt. Vorzugsweise wird zur Stabilsierung Ascorbinsäure in 4fach molarem Überschuß in Bezug auf das Hämoglobin eingesetzt.

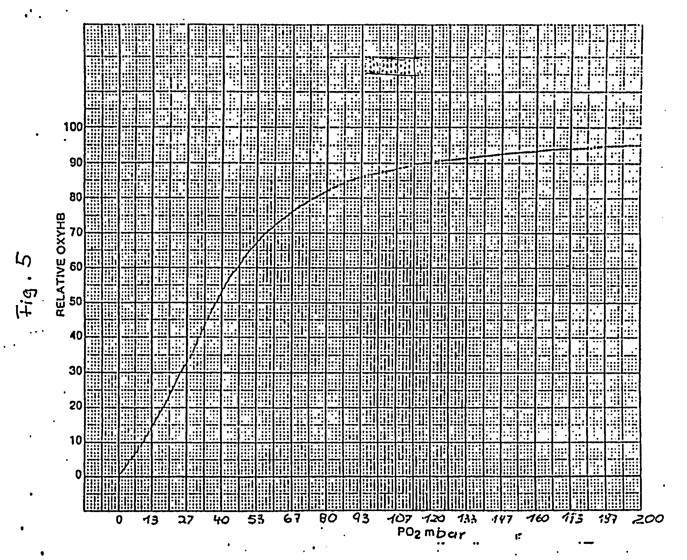

Die Sauerstoffbindungskurve der erfindungsgemäß hergestellten vernetzten Hämoglobinlösung unter Verwendung von Pyridoxalphosphat ist in Fig. 5 dargestellt. Der $p_{50}$-Wert beträgt 36 mbar. Überraschenderweise zeigt die Kurve einen sigmoiden, also dem nativen Hämoglobin ähnlichen Verlauf. Ein solches Verhalten wurde bisher bei vernetzten Hämoglobinen noch nicht gefunden.

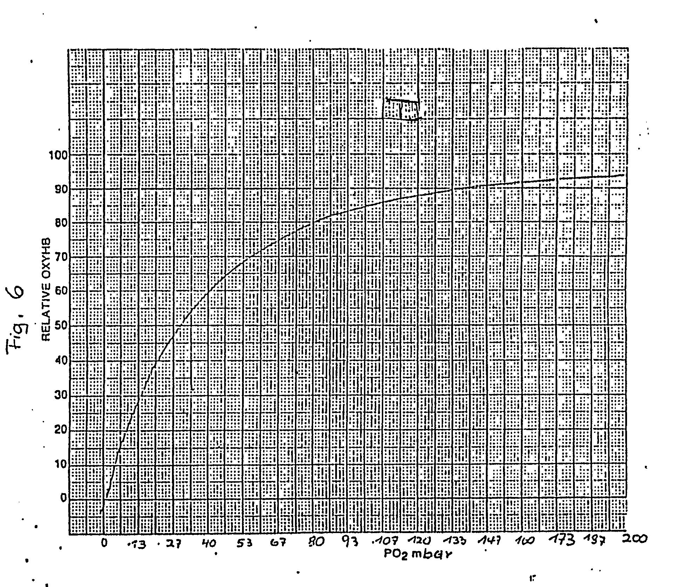

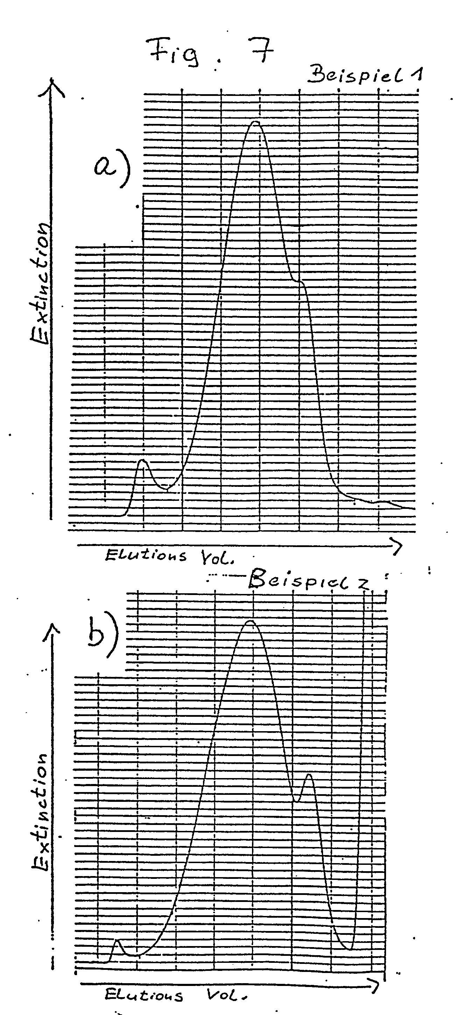

Die Sauerstoffbindungskurve unter Verwendung von Inosithexaphosphat ist in Fig. 6 dargestellt.

Sowohl die Wirksamkeit als auch die Verträglichkeit der nach dem erfindungsgemäßen Verfahren hergestellten Hämoglobinlösungen wurden tierexperimentell an Kaninchen und Ratten nachgewiesen. Die nach dem erfindungsgemäßen Verfahren hergestellten Präparate waren pyrogenfrei.

Nachstehende Beispiele dienen der weiteren Erläuterung der Erfindung.

## Beispiel 1

Eine gemäß Beispiel 1 der DE-PS 22 48 475 hergestellte Hämoglobinlösung wurde durch Ultrafiltration mittels einer Hollow-Fiber-Patrone mit einer Durchlässigkeit bis MG 100 000 D auf eine Konzentration von 19,6% gebracht. Zu dieser Lösung fügte man in 4fach molarem Überschuß neutralisierte Ascorbinsäurelösung zu, sterilfiltrierte und ließ mindestens 24 Stunden stehen. 842 ml dieser desoxygenierten, 19,6%igen Hämoglobinlösung wurden vorgelegt, gekühlt und mit 4,3 g $NaH_2PO_4$ sowie 3 g $Na_2HPO_4$, in wenig Wasser gelöst, versetzt.

Nun wurden unter Rühren 2,2 g Pyridoxalphosphat zugefügt. Der PH-Wert sank auf 6,6 ab. Die Reaktionszeit betrug eine Stunde. Nach dieser Zeit wurden unter kräftigem Rühren 13,9 ml 10%ige wäßrige Glutardialdehydlösung hinzugegeben und wiederum 1 Stunde gerührt. Zur anschließenden Reduktion fügte man 1,34 g $NaBH_4$ zu, das kurz vorher in wenig Wasser gelöst wurde.

Nach einer Reaktionszeit von 30 Minuten verdünnte man die stark schäumende Lösung mit 6 l Wasser, wobei der Schaum größtenteils zusammenfiel, und filtrierte nach 1stündiger Behandlung mit 10 g/l Aktivkohle diese Lösung über ein Sterilfilter. Zur anschließenden Ultrafiltration mit der Hollow-Fiber-Patrone mit einer Durchlässigkeit bis MG 100 000 D füllte man zunächst das Ultrafiltrationssystem und legte dann 1,1 l der verdünnten Lösung in einem geschlossenen Gefäß vor. Diese Menge wurde ständig im Kreislauf gepumpt. Der durch das Ultrafiltrat bedingte Volumenverlust wurde ständig durch die verdünnte Hämoglobinlösung aufgefüllt. Auf diese Weise wurde die Lösung wieder ankonzentriert. Sobald die Hämoglobinlösung zur Substitution des Volumenverlustes verbraucht war, wurde die Ultrafiltration mit 9 l destilliertem Wasser fortgesetzt und anschliessend auf ca. 10 bis 11 % aufkonzentriert. Zur Einstellung des kolloidosmotischen Druckes und der Hämoglobinkonzentration fügte man 115 ml 20%iges Humanalbumin zu.

Vor der abschließenden Sterilfiltration wurden noch folgende Inhaltsstoffe dazugegeben:

3,32 g NaCl, 14,5 g Glukose, 1,66 g $NaHCO_3$, 0,26 g KCl, 0,16 g $MgSO_4$ und zur Stabilisierung 0,61 g neutralisierte Ascorbinsäure.

Ausbeute: 660 ml
Hb-Gehalt: 8,5 %
Met-Hb: 5,1 rel. %
Kolloidosmotischer Druck 36,8 mbar
$P_{50}$-Wert 38,6 mbar
relative Viskosität 2,6
Molekulargewichtsverteilung siehe Fig. 7a.
(gelchromatographisches Profil über Sepharose® 6B)

## Beispiel 2

Man verfuhr wie in Beispiel 1. Statt des Pyridoxalphosphats wurden als Effektor 2,59 g Inosithexaphosphat, in Wasser gelöst und auf pH 7,6 eingestellt zugegeben.

Ausbeute: 925 ml

ɔilung siehe Fig. 7b.
es Profil über Sepharose 6B)

erstellung eines lagerstabilen, vernetzten Hämoglobinpräparates mit hoher
ität, bei dem man eine stromafreie Hämoglobinlösung deoxygeniert und mit einem
gruppen-spezifischen Reduktionsmittel und bei einem pH-Wert zwischen 6 und 8 mit
:offketten mit 3 bis 8 C-Atomen versetzt,
ɪet, daß man vor dem Zusatz des Effektors eine stromafreie angereicherte
ɔoviel eines Sauerstoff verbrauchenden Reduktionsmittels versetzt, daß der $O_2$-
ɪnkt, das nach Zusatz des Effektors erzielte Produkt anschließend mit dem Dialdehyd
rnetzte Produkt mit der 5- bis 20-fachen Menge des carbonylgruppen-spezifischen
ɘrt, die Lösung mit Wasser verdünnt, ggf. mit Aktivkohle behandelt, solange im
der Gehalt an unvernetztem Hämoglobin höchstens noch 15 % beträgt, wobei die
ährend der Ultrafiltration erfolgen kann, und das Produkt durch Zugabe eines
ɪert.
ɔpruch 1, dadurch gekennzeichnet, daß man als Effektor Pyridoxalphosphat oder
ɘndet.
ɔpruch 1 oder 2, dadurch gekennzeichnet, daß man als Sauerstoff verbrauchendes
ɔierte Ascorbinsäure verwendet.
ɘinem der vorstehenden Ansprüche, dadurch gekennzeichnet, daß man als
ɔhes Reduktionsmittel $NaBH_4$ verwendet.
ɪztes Hämoglobinpräparat mit hoher Sauerstoff-Transportkapazität, erhalten durch
ɘm der vorstehenden Ansprüche.

aration of a crosslinked hemoglobin of extended shelf life and high oxygen transport
a-free hemoglobin solution is deoxygenated and treated with an effector, a carbonylagent and at a pH between 6 and 8 with a dialdehyde having carbon chains of 3 to 8
zed by treating a stroma-free, enriched hemoglobin solution prior to the addition of
ɪ of an oxygen-consuming reducing agent that the $O_2$ partial pressure diminishes to 0
ɪroduct obtained after the addition of the effector with the dialdehyde, reducing the
ɔtained with 5 to 20 times the amount of the carbonyl-group-specific reducing agent,
water, ultrafiltering and recirculating the solution until the content of un-crosslinked
ɔ more than 15%, the crosslinking being effected before or during the ultra-filtration,
ɘnt thereby to stabilize the product.
ɔ claim 1, characterized in that pyridoxal phosphate or inositol hexaphosphate are

to claim 1 or 2, characterized in that neutralized ascorbic acid is used as
ɪg agent.
ɔ one of the preceding claims, characterized in that $NaBH_4$ is used as carbonyl-
ɪent.
ɔin preparation of long shelf life and high oxygen transport capacity produced by the
ɘding claims.

ion d'une préparation d'hémoglobine réticulée, stable à la conservation, ayant une
ɔrt d'oxygène, dans lequel on désoxygène une solution d'hémoglobine dépourvue de
ɘc un effecteur, un réducteur spécifique des groupes carbonyle et à un pH compris
éhydes ayant des chaînes carbonées en C3 à C8, caractérisé en ce qu'avant l'addition
une solution d'hémoglobine concentrée dépourvue de stroma avec suffisamment de
ɘ l'oxygène pour que la pression partielle d'oxygène tombe à 0 mbar, en ce qu'on
dialdéhyde le produit obtenu après addition de l'effecteur, en ce qu'on réduit le

produit ainsi réticulé avec une quantité 5 à 20 fois supérieure du réducteur spécifique des groupes carbonyle, en ce qu'on dilue la solution avec de l'eau, en ce qu'on traite le cas échéant avec du charbon actif, en ce qu'on procède à une ultrafiltration dans la circulation jusqu'à ce que la teneur en hémoglobine non réticulée ne s'éléve plus qu'à 15% au plus, la réticulation pouvant s'effectuer avant ou après l'ultrafiltration, et en ce qu'on stabilise le produit par addition d'un réducteur.

2. Procédé selon la revendication 1, caractérisé en ce qu'on utilise comme effecteur le phosphate de pyridoxal du l'hexaphosphate d'inositol.

3. Procédé selon les revendications 1 et 2, caractérisé en ce qu'on utilise comme réducteur consommant de l'oxygène, de l'acide ascorbique neutralisé.

4. Procédé selon l'une des revendications précédentes, caractérisé en ce qu'on utilise comme réducteur spécifique des groupes carbonyle $NaBH_4$.

5. Préparation d'hémoglobine réticulée, stable à la conservation, ayant une capacité élevée de transport d'oxygène, obtenue par le procédé selon l'une des revendications précédentes.

Fig. 1

Fig. 2

# Molekulargewichtsverteilungen Fig. 3

nach Herstellung

5 Wochen nach Herstellung

Fig. 4

Molekulargewichtsverteilungen

nach Reduktion mit $NaBH_4$

a) nach Herstellung

b) 10 Wochen nach Herstellung

Fig. 5

Fig. 6

0078961

Fig. 7

Beispiel 1

a)

Extinction

Elutions Vol.

Beispiel 2

b)

Extinction

Elutions Vol.

13